# EUROPÄISCHE PATENTSCHRIFT

(11) **EP 0 604 495 B1**
(45) Veröffentlichungstag und Bekanntmachung des Hinweises auf die Patenterteilung: **30.07.1997**
(21) Anmeldenummer: 92919286.2
(22) Anmeldetag: 15.09.1992
(51) Int. Cl.: C12N 15/52, C12Q 1/68, C12N 15/54, C12N 15/61, C12P 21/02, C12P 19/62

(54) **SEKUNDÄRMETABOLIT-BIOSYNTHESEGENE AUS AKTINOMYCETEN, VERFAHREN ZU IHRER ISOLIERUNG SOWIE IHRE VERWENDUNG**
SECONDARY-METABOLITE BIOSYNTHESIS GENES FROM ACTINOMYCETES, METHOD OF ISOLATING THEM, AND THEIR USE
GENES DE BIOSYNTHESE DE METABOLITES SECONDAIRES OBTENUS A PARTIR D'ACTINOMYCETES, PROCEDE D'ISOLEMENT DESDITS GENES, ET LEUR UTILISATION

(30) Priorität: 18.09.1991 DE 4130967
(43) Veröffentlichungstag der Anmeldung: 06.07.1994
(73) Patentinhaber: HOECHST AKTIENGESELLSCHAFT, 65926 Frankfurt am Main (DE)
(72) Erfinder: PIEPERSBERG, Wolfgang, D-5600 Wuppertal 12 (DE); STOCKMANN, Michael, D-5300 Bonn (DE); TALEGHANI, Kampiz, Mansouri, D-8000 München 80 (DE); DISTLER, Jürgen, D-5600 Wuppertal 1 (DE); GRABLEY, Susanne, D-6240 Königstein (DE); SICHEL, Petra, D-6090 Rüsselsheim (DE); BRÄU, Barbara, D-6230 Frankfurt am Main 80 (DE)
(86) Internationale Anmeldenummer: EP9202111
(87) Internationale Veröffentlichungsnummer: WO9306219

(56) Entgegenhaltungen:
- WO-A-87/03907
- FEMS MICROBIOLOGY LETTERS, Bd. 90, Nr. 2, 1. Januar 1992, AMSTERDAM, THE NETHERLANDS, Seiten 185-190; M. STOCKMANN ET W. PIEPERSBERG: 'Gene probes for the detection of 6-deoxyhexose metabolism in secondary metabolite-producing streptomycetes'

## Beschreibung

Die Erfindung betrifft Sekundärmetabolit-Biosynthesegene aus Streptomyces nodosus DSM 40109 sowie deren codierende DNA-Sequenz.

Eines der Arbeitsgebiete der Gentechnologie ist die Isolierung bestimmter Gene unmittelbar aus dem Genom. Um das zu isolierende Gen aufzufinden, kann man z.B. Gensonden einsetzen, die spezifisch an die gewünschte DNA-Sequenz binden. Damit kann man diese aus einer großen Anzahl anderer Sequenzen "herausfischen" (screenen).

Bisher untersuchte Sekundärmetabolit-Biosynthesegene (Gene für Antibiotika, Anthelmintika, antifungische Stoffe, Enzyminhibitoren, Farbstoffe etc.) liegen benachbart innerhalb einer Einheit auf dem bakteriellen Chromosom oder auf sehr großen Plasmiden vor. Dies gilt insbesondere für Streptomyceten und andere Aktinomyceten [C. L. Hershberger et al. (1989), Genetics and Molecular Biology of Industrial Microorganisms, Am. Soc. for Microbiol., Washington, D.C. 20005, S. 35 - 39, S. 58, S. 61 - 67, S. 147 - 155].

So befinden sich z.B. 6-Desoxyzucker-Biosynthesegene von Aktinomyceten in enger Nachbarschaft zu anderen Sekundärmetabolit-Biosynthesegenen [J.F. Martin et al. (1989), Ann. Rev. Microbiol 43: 173-206] auf dem Genom.

Es ist darüberhinaus bekannt, daß eine Vielzahl von Sekundärmetaboliten aus Aktinomyceten 6-Desoxyzuckerreste enthalten. Beispiele sind u.a. Aminoglykoside (z.B. Spektinomycin, Kasugamycin, Streptomycin), Polyene (z.B. Amphotericin A, B, Nystatin), Macrolide (z.B. Tylosin, Erythromycin, Avermectin), Nukleoside (z.B. Antibiotikum A201A) und Anthrazykline (z.B. Daunorubicin, Cytorhodin A) und Glykopeptide (z.B. Vancomycin), Isochromanchinone (z.B. Granaticin).

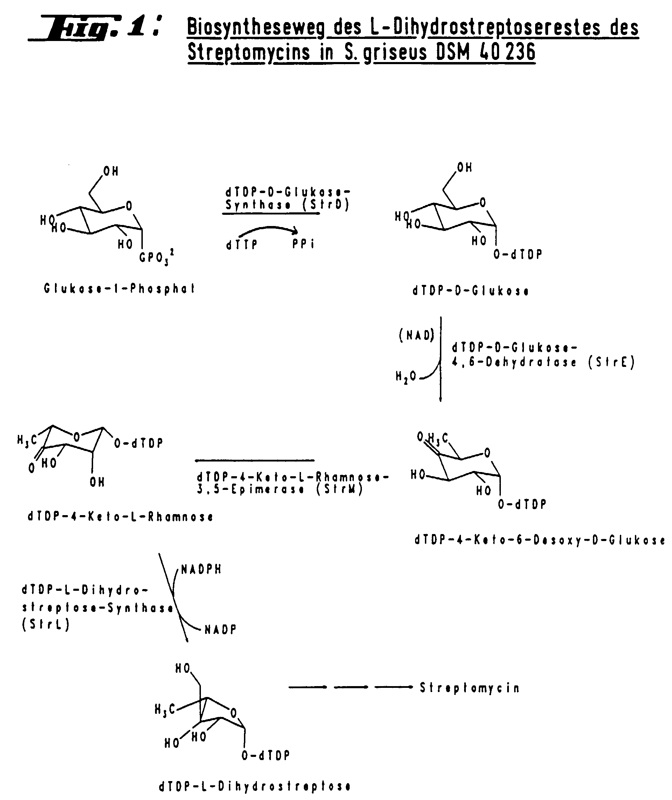

Der Biosyntheseweg eines 6-Desoxyzuckerrestes des Streptomycins, des L-Dihydrostreptoserestes, ist in Abb. 1 dargestellt.

Bis vor kurzem waren noch keine Sequenzdaten von Genen oder Enzymen aus Aktinomyceten vorhanden, die an der Biosynthese von 6-Desoxyzuckern beteiligt sind. Durch Klonierung und Analyse der Streptomycin-Biosynthesegene konnten die Gene für die 6-Desoxyzucker-Komponente L-Dihydrostreptose isoliert und identifiziert werden:
strD (dTDP-D-Glukose-Synthase), strE (dTDP-D-Glukose-4,6-Dehydratase), strM (dTDP-4-Keto-L-Rhamnose-3,5-Epimerase) und strL (dTDP-L-Dihydrostreptose-Synthase) aus Streptomyces griseus DSM40236.

Der Einsatz heterologer Gensonden, d.h. Gensonden, die für das Screening in einer anderen Species oder für die Isolierung von Genen innerhalb eines anderen Biosynthesewegs eingesetzt werden, ist für die Isolierung von Sekundärmetabolit-Biosynthesegenen bisher auf wenige Gene (z.B. Polyketidsynthetasegene) beschränkt [Nature (1987) 325: 818-821]. Diese Polyketidsynthetase-Gensonden erlauben lediglich das Auffinden von Verbindungen, die über den Polyketidsynthetase-Biosyntheseweg gebildet werden [C.L. Hershberger et al. (1989), Genetics and Molecular Biology of Industrial Microorganisms, Am. Soc. for Microbiol., Washington, D.C. 20005, S. 76-78; S.L. Otten et al., J. Bacteriol. 172, No. 6 (1990). S. 3427-3434], aber nicht das Auffinden von funktionell andersartigen Genen, wie z.B. Aminoglykosid-Biosynthesegenen.

Überraschend wurde nun gefunden, daß sich ein oder mehrere Gensonden aus der Gruppe der Gene strD, strE, strL oder strM aus Streptomyces griseus DSM40236 sowie strukturell verwandte Gene als Gensonden für das Auffinden der Gene snoT (kodierend für Amphotheronolid B-dTDP-D-Mycosaminyl-Transferase), snoD (kodierend für dTDP-D-Glukose-Synthase) und snoM (kodierend für dTDP-4-Keto-6-Desoxy-D-Glukose-lsomerase) oder einem oder mehreren Sekundärmetabolit-Biosynthesegenen aus Aktinomyceten eignen. Das Screening nach den Sekundärmetabolit-Biosynthesegenen erfolgt unabhängig von der chemischen Struktur der Sekundärmetabolite. Voraussetzung ist lediglich, daß sie 6-Desoxyzuckerreste besitzen.

Die Erfindung offenbart daher:

Die vollständige DNA-Sequenz von snoT und snoD sowie die DNA-Teilsequenz von snoM.

Die vollständige Aminosäuresequenz von snoT und snoD sowie die Aminosäureteilsequenz von snoM.

Ein Verfahren zur Isolierung von Sekundärmetabolit-Biosynthesegenen aus Aktinomyceten, dadurch gekennzeichnet, daß ein oder mehrere Gene aus der Gruppe der Gene strD, strE, strL oder strM aus Streptomyces griseus DSM40236 sowie strukturell verwandte Gene als Gensonden für das Auffinden der Gene snoT (kodierend für Amphotheronolid B-dTDP-D-Mycosamin-Transferase), snoD (kodierend für dTDP-D-Glukose-Synthase) und snoM (kodierend für dTDP4-Keto-6-Desoxy-D-Glukose-lsomerase) oder einem oder mehreren Sekundärmetabolit-Biosynthesegenen aus Aktinomyceten verwendet werden.

Die Erfindung wird im folgenden detailliert beschrieben.
Ferner wird die Erfindung durch den Inhalt der Ansprüche bestimmt.

Alle gentechnologisch dargestellten Methoden wurden, wenn nicht anders angegeben, J. Sambrook et al. (Molecular Cloning; A laboratory manual [2nd edition] 1989; Cold Spring Harbour Laboratory Press, N.Y., USA) entnommen.

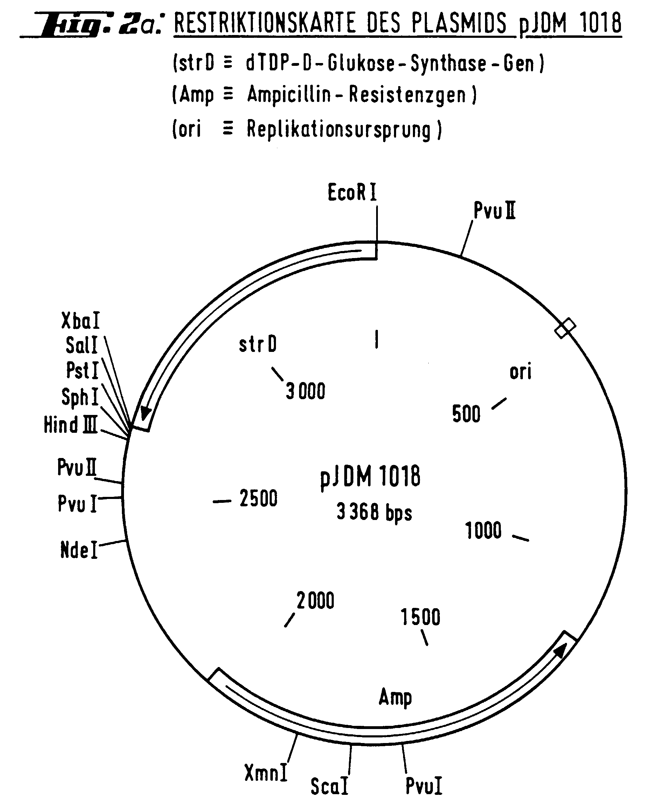

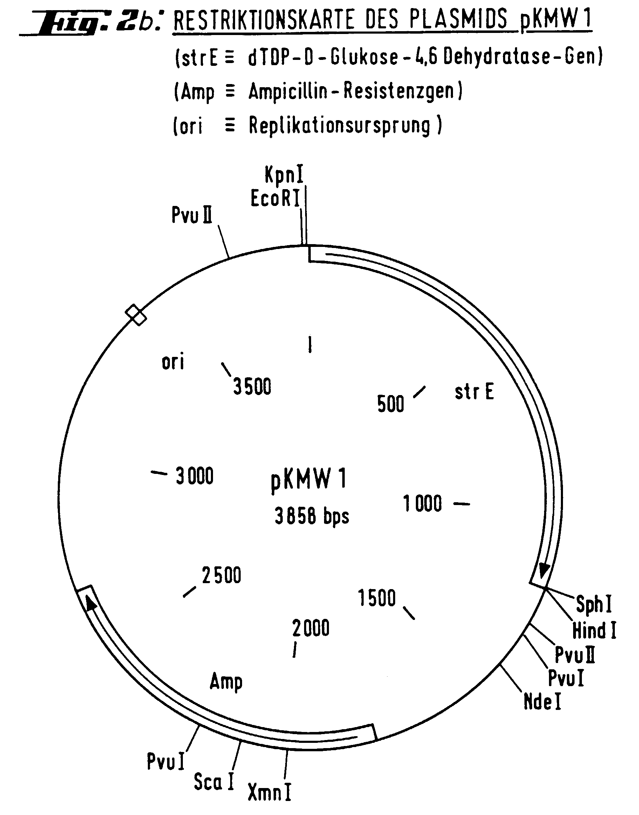

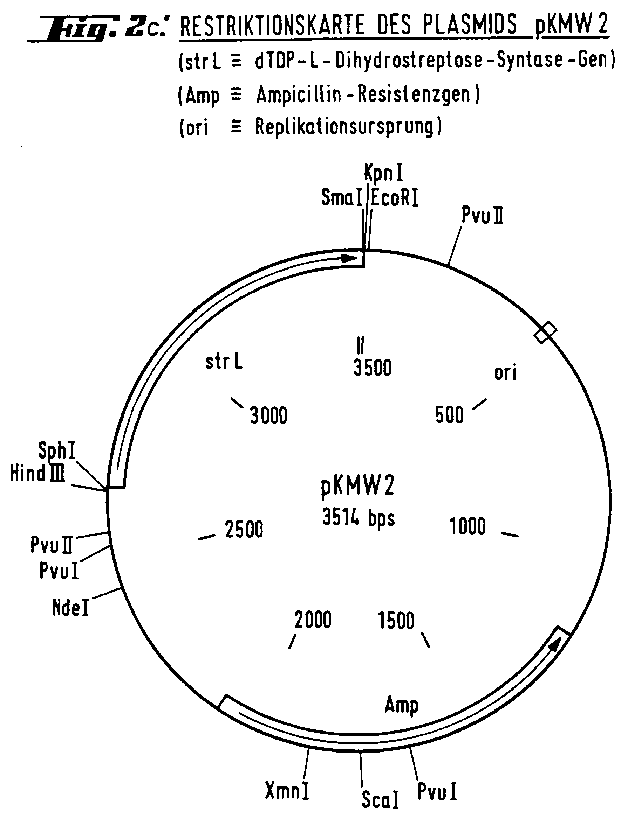

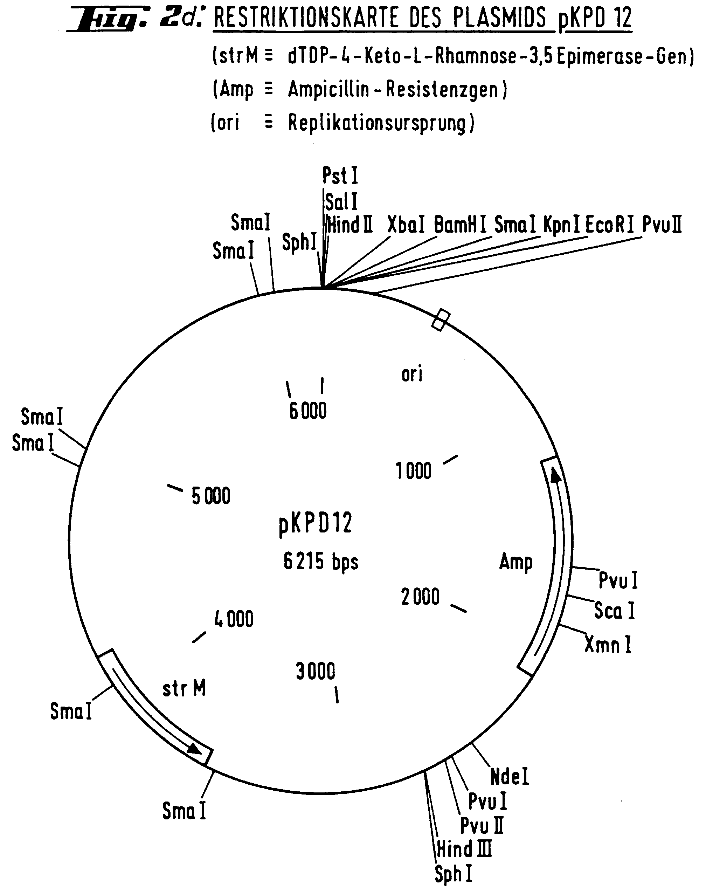

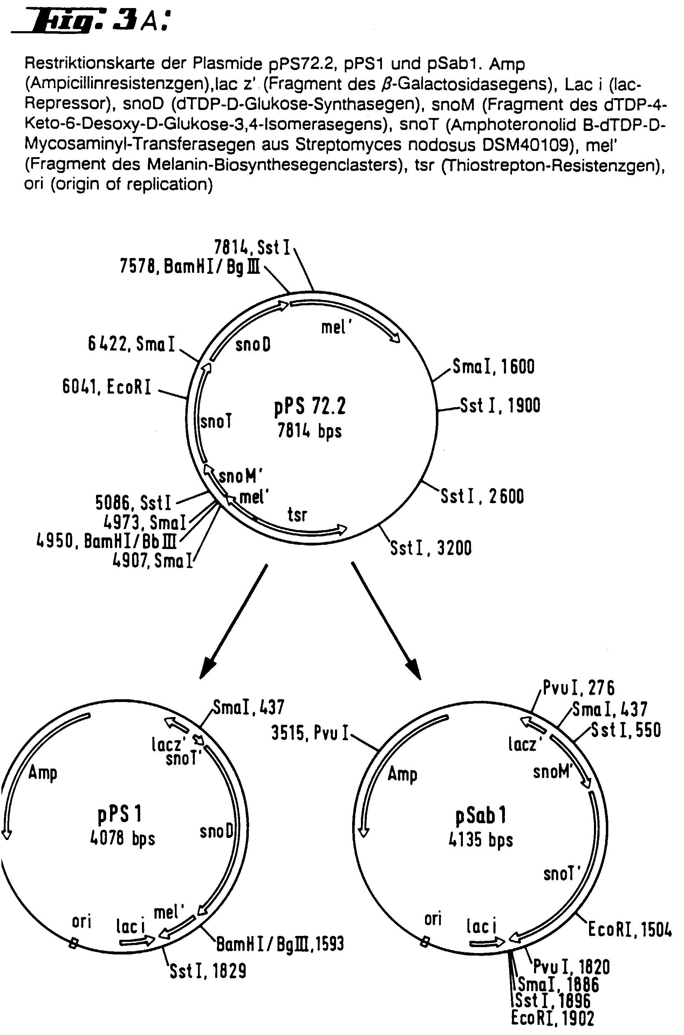

Die für das Screening notwendigen Gensonden strD, strE, strL und strM sind in den E. coli Stämmen FH-L 8138 (strD), FH-L 8154 (strE), FH-L 8158 (strL) und FH-L 8159 (strM) bei der Deutschen Sammlung von Mikroorganismen und Zellkulturen GmbH (DSM) am 30.08.1991 in 3300 Braunschweig, Mascheroder Weg 1b, Deutschland, unter folgenden Nummern DSM6681 (FH-L 8138), DSM6682 (FH-L 8154), DSM6683 (FH-L 8158), DSM6684 (FH-L 8159), hinterlegt. Die Plasmide pJDM1018 (strD), pKMW1 (strE), pKMW2 (strL) und pKPD12 (strM) [siehe Abb. 2] werden aus den obengenannten E. coli-Stämmen mit Hilfe der "Boiling-Method" oder durch alkalische Lyse isoliert (J. Sambrook et al. 1989).

Die Plasmide pJDM1018 (strD) aus E. coli DSM6681, pKMW1 (strE) aus E. coli DSM6682 werden mit den Restriktionsenzymen EcoRI und Hind III geschnitten. Ein 0,7 kb EcoRI/Hindlll-Fragment des Plasmids pJDM1018 bzw. ein 1,2 kb EcoRI/Hindlll Fragment des Plasmids pKMW1 wird isoliert und durch sogenannte "Nick-translation" mit ³²P-markierten Desoxynukleotiden versehen. Diese radioaktiv markierten Fragmente werden im folgenden als strD- bzw. strE-Gensonden bezeichnet.

Aus dem Plasmid pKMW2 (strL) aus E. coli DSM6683 wird ein 0,8 kb EcoRI-HindIII-Fragment, bzw. aus dem Plasmid pKPD12 (strM) aus E. coli DSM6684 ein 0,5 kb Smal-Fragment isoliert, radioaktiv markiert und als strL- bzw. strM-Gensonde eingesetzt.

Grundsätzlich kann anstelle der obengenannten Gensonden auch jedes verlängerte, verkürzte oder modifizierte Fragment bzw. synthetisches Oligodesoxynukleotid als Gensonde verwendet werden, solange es mit dem Gen für die Biosynthese von Desoxyzucker-Resten hybridisiert.

Für die Isolierung eines oder mehrerer Sekundärmetabolit-Biosynthesegene geht man wie folgt vor:

Die Gesamt-DNA eines jeden in Tabelle 1 aufgeführten Aktinomyceten-Stämme kann isoliert werden, mit der Restriktionsendonuklease BamHI und anderen Restriktionsenzymen gespalten und gelchromatographisch aufgetrennt werden. Vorzugsweise verwendet man die Gesamt-DNA von Streptomyces nodosus DSM40109.

Anschließend erfolgt die Hybridisierung mit 6-Desoxyzucker-Gensonden, vorzugsweise mit der strD-Gensonde. Die DNA-Fragmente, vorzugsweise die Fragmente in der Größe von 1,5 - 3 kb, die mit den 6-Desoxyzucker-Gensonden hybridisieren, vorzugsweise mit der strD-Gensonde aus S. nodosus DSM40109, werden aus dem Gel isoliert und in einen geeigneten Vektor ligiert und in einem für den Vektor kompatiblen Mikroorganismus kloniert.

Bevorzugt werden die Vektoren pUC18 (Boehringer Mannheim, Deutschland) und pEB15 aus S. coelicolor Müller DSM4914 sowie die Wirtsstämme E. coli K12 und S. lividans 66 eingesetzt. Ganz bevorzugt wird E. coli DH5Alpha (Gibco BRL, Eggenstein, Deutschland) und S. sp DSM40434 verwendet.

Die Plasmid-DNA enthaltenden Klone werden isoliert und mit der jeweiligen Gensonde, bevorzugt mit der strD-Sonde aus S. griseus DSM40236, hybridisiert. Dabei können jeweils Zehnerpools verwendet werden, welche im Fall einer Hybridisierung anschließend in die Einzelklone ausgesplittet werden, die Plasmide isoliert und erneut für die Hybridisierung eingesetzt. Ein Plasmid, welches mit einer Gensonde hybridisiert, wird durch Restriktionsenzyme gespalten. Hybridisierende Restriktionsfragmente, vorzugsweise solche, die mit der strD-Gensonde aus S. Nodosus DSM40109 hybridisieren, werden isoliert und in einen Vektor, bevorzugt pUC18, in einen Wirtsstamm, bevorzugt E. coli DH5Alpha, subkloniert und durch DNA-Sequenzanalyse untersucht.
Gegebenenfalls kann durch erneute Subklonierung vor der DNA-Sequenzanalyse der zu sequenzierende Bereich des klonierten Fragments näher begrenzt werden und für die Sequenzierung geeignete Subklone durch Hybridisierung, vorzugsweise mit der strD-Gensonde, identifiziert werden.

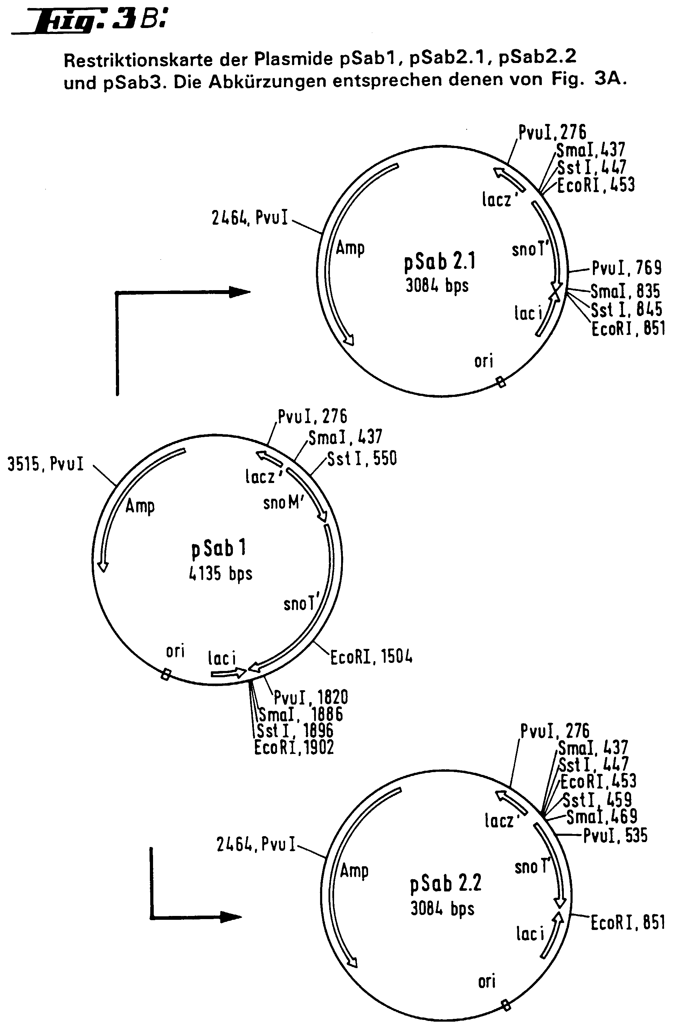

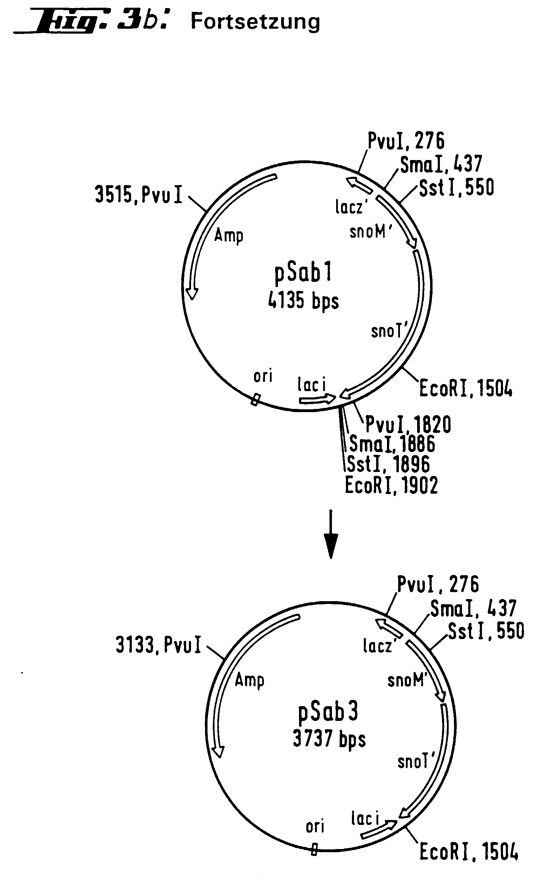

Bevorzugt wird das aus der Gesamt-DNA des Amphotericin B-Produzenten S. nodosus DSM40109 isolierte 2,6 kb BamHI-Fragment, welches mit der strD-Sonde hybridisiert, in den Vektor pEB15 in S. sp. DSM40434 kloniert. Das erhaltene Plasmid pPS72.2 (s. Abb. 3A) wird mit Restriktionsenzymen gespalten, z.B. mit Smal-Sstl, und die entstehenden DNA-Fragmente in pUC18 in E. coli DH5Alpha subkloniert und sequenziert.

Für die Subklonierung werden die obengenannten DNA-Fragmente isoliert. Bevorzugt wird das 1,4 kb Smal/Sstl-, das 1,45 kb Smal-Fragment aus dem Plasmid pPS72.2 isoliert und in pUC18 subkloniert, wobei die Plasmide pPS1 und PSab1 entstehen (Abb. 3A). Bevorzugt wird das 0,4 kb EcoRI-Fragment aus dem Plasmid pSab1 isoliert und in beiden möglichen Orientierungen in pUC18 in E. coli DH5Alpha kloniert, wobei die Plasmide pSab2.1 und pSab2.2 entstehen (s. Abb. 3A), das 3,7 kb EcoRI-Fragment des Plasmids pSabl wird isoliert, religiert und in E. coli DH5Alpha transformiert, wobei das Plasmid pSab3 entsteht (s. Abb. 3A).

Die Plasmide, welche ein mit einer Gensonde hybridisierendes DNA-Fragment enthalten, werden für die DNA-Sequenzierung eingesetzt. Bevorzugt wird das in dem Plasmid pPS72.2 enthaltene 2,6 kb BamHI-Fragment von S. nodosus DSM40109 vollständig und doppelsträngig sequenziert. Dazu werden bevorzugt das Plasmid pPS72.2 und die davon abgeleiteten Plasmide pPS1, pSabl, pSab3, pSab2.1 und pSab2.2 verwendet, die zuvor durch alkalische Lyse isoliert und durch zweimalige Cäsiumchlorid-Gradienten-Zentrifugation gereinigt werden.

Für die DNA-Sequenzierung wird die Methode nach A.M. Maxam und W. Gilbert (1977) Proc. Natl. Acad. Sci. USA 74:560-564 oder F. Sanger et al. (1977) Proc. Natl. Acad. Sci. USA (1977) 74:5463-5467 oder ein davon abgeleitetes Verfahren angewandt. Bevorzugt wird das Promega fmol™ Sequencing System verwendet, welches nach der Methode von Sanger (Serva, Heidelberg, Deutschland) arbeitet.

Geeignete Primer werden käuflich erworben (pUC reverse sequencing und sequencing Primer, Boehringer Mannheim, Deutschland) oder wie in der europäischen Offenlegungsschrift 0 368 244 (Beispiel 16) beschrieben synthetisiert (s. Tabelle 2).

Offene Leseraster oder Teile davon werden an der für Streptomyceten charakteristischen Codon-Verwendung (F. Wright et al. 1992, Gene 113:55-65) und am Vorhandensein eines Startcodons (ATG oder GTG) und/oder eines Stopcodons (TAG, TGA oder TAA) erkannt. Aus der DNA-Sequenz abgeleitete Aminosäuresequenzen werden mit der Aminosäureabfolge bekannter Genprodukte verglichen. Bevorzugt wird die Analyse offener Leserahmen mit dem FASTA- oder TFASTA-Programm des Database Searching Programms, Version 7, des GCG-Package, Genetics Computer Group Inc. Wisconsin, USA) verwendet. Dabei wird eine oder mehrere der Datenbanken SwissProt, NBRF-Protein (Pir), Genbank und/oder EMBL bezüglich einer Sequenzähnlichkeit mit dem identifizierten offenen Leserahmen untersucht. Aus dem Aminosäuresequenzvergleich mit bekannten Proteinen und einem Vergleich mit dem angenommenen Biosyntheseweg für dTDP-D-Mycosamin (Juan-Francisco Martin, Biosynthesis of Polyene Macrolide Antibiotics; Ann. Rev. Microbiol. 1977, 31:13-38)wird auf die Funktion des jeweiligen Genproduktes geschlossen.

Aternativ können auch die DNA-Sequenzen mit bekannten DNA-Sequenzen und entsprechenden Leserahmen verglichen werden.

Vorzugsweise werden die Genprodukte, welche als snoM, snoT und snoD bezeichnet werden und für die dTDP-4-Keto-6-Desoxy-D-Glukose-3,4-Isomerase, die B-dTDP-Mycosaminyl-Transferase und die dTDP-D-Glukose-Synthase kodieren, identifiziert.

Die identifizierten Gene snoM, snoT und snoD können außerdem zum Auffinden neuer 6-Desoxyzucker-hatiger Sekundärmetabolite eingesetzt werden.

Für das Auffinden von neuen Sekundärmetabolit- und 6-Desoxyzucker-Biosynthesegenen aus Aktinomyceten eignen sich alle 6-Desoxyzucker-Biosynthesegene aus Aktinomyceten als Gensonden, besonders jedoch die L-Dihydrostreptose-Biosynthesegene strL und strM und ganz vorzugsweise die Gene strD und strE aus Streptomyces griseus DSM40236.

Anstelle der strD, strE, strL und strM Gensonden aus Streptomyces griseus DSM40236 können auch funktionell und strukturell ähnliche Gene als Sekundärmetabolit-spezifische Sonden eingesetzt werden, so z.B. aus dem Hydroxystreptomycinproduzenten Streptomyces glaucescens DSM40716, den Candicidinproduzenten Streptomyces coelicolor DSM40624 und Streptoverticillium sp. DSM40237, dem Perimycinproduzenten Streptomyces aminophilus DSM40186, dem Pimaricinproduzenten Streptomyces sp. DSM40357, dem Lucensomycinproduzenten Streptomyces lucensis DSM40317, dem Rimocidinproduzenten Streptomyces rimosus DSM40260, dem Levorin A2-Produzenten Streptomyces sp. DSM40202, dem Lienomycinproduzenten Streptomyces lienomycini ATCC43687, dem Monazomycinproduzenten Streptoverticillium mashuense NRRL B-3352, den Pikromycinproduzenten Streptomyces felleus DSM40130 und Streptomyces olivaceus DSM40702, dem Narbomycinproduzenten Streptomyces narbonensis DSM40016, den Methymycinproduzenten Streptomyces venezuelae ATCC15068 und ATCC15439 eingesetzt werden.

Vorzugsweise werden die Gene aus dem Amphotericin B Produzenten Streptomyces nodosus DSM40109, dem Nystatinproduzenten Streptomyces noursei DMS40635, dem Rhodomycinproduzenten Streptomyces purpurascens DSM2658 und dem Streptomycinproduzenten Streptomyces griseus DMS40236 verwendet.

Die sekundärmetabolit-spezifischen Gensonden können außerdem verwendet werden für:
- die Bildung hybrider Naturstoffe:
   Hierfür bringt man mit Hilfe der genannten Gensonden isolierte Gene in einen anderen Aktinomyceten-Stamm, um zu erreichen, daß dieser Stamm einen neuartigen Sekundärmetaboliten synthetisiert. Hierzu sind insbesondere die 6-Desoxyzucker-Biosynthese- und Transferase-Gene, aber auch andere Sekundärmetabolit-Biosynthesegene zur Bildung neuartiger hybrider Naturstoffe geeignet.
- die Steigerung der Sekundärmetabolitausbeute in Aktinomyceten:
   In einigen Aktinomyceten-Stämmen ist die Aktivität der 6-Desoxyzucker-Biosyntheseenzyme durch unterschiedliche Faktoren limitiert. Durch Klonierung der 6-Desoxyzucker-Biosynthesegene aus einem Aktinomyceten-Stamm und Wieder-Einbringung des klonierten Gens in höherer Kopienzahl in diesen Stamm kann die Ausbeute an Genprodukten und damit eine Erhöhung der Sekundärmetabolitproduktion erreicht werden. Gleiches gilt für andere, klonierte Sekundärmetabolit-Biosynthesegene.
- die Gewinnung von Biosyntheseenzymen:
   Die chemische Synthese von 6-Desoxyzuckern ist aufwendig, da sie eine ausgefeilte Schutzgruppentechnik erfordert. Vorteilhaft ist die Schutzgruppenfreie, in vitro Synthese der Zucker mit Hilfe von Enzymen.
   Liegen die oben beschriebenen 6-Desoxyzucker-Biosynthesegene in erhöhter Kopienzahl vor, ist die Bereitstellung der 6-Desoxyzucker-Biosynthese-Enzyme für die enzymatische Synthese erleichtert.
- die Biotransformation in Aktinomyceten:
   Die Glykosylierung eines Sekundärmetabolit-Vorläufers (natürliches Aglykon) in einem Aktinomycetenstamm wird durch 6-Desoxyzucker-Biosynthese- und Transferasegenprodukte bewirkt.
   Andere Verbindungen (fremde Aglyka) können einem Stamm zugefüttert und glykosyliert werden. Durch Selbstklonierung, d.h. Klonierung aus einem Stamm und Wiedereinführung in hoher Kopienzahl in denselben Stamm, von 6-Desoxyzucker-Biosynthese- und Transferasegenen wird die Glykosylierungsrate eines fremden (zugefütterten) Aglykons erhöht.
- Identifizierung anderer Gene:
   Wie eingangs schon erwähnt, können mit Hilfe von Gensonden auch strukturell und funktionell neuartige Gene isoliert werden, für welche bisher keine Sequenzhomologie bekannt ist.
- Screening von Sekundärmetabolit-Produzenten:
   Mit Hilfe der obengenannten Gensonden werden 6-Desoxyzucker-haltige Sekundärmetabolit-Produzenten identifiziert. Dies führt wiederum zur Isolierung von neuartigen Sekundärmetabolite.

### Beispiel

### 1. Anzucht der E. coli Stämme, Präparation von Plasmid-DNA und Isolierung von DNA-Fragmenten

Die Stämme E. coli DSM6681 (enthält das strD Gen auf Plasmid pJDM1018) und E. coli DSM6682 (enthält das strE Gen auf Plasmid pKMW1) werden jeweils in einem Liter Luria Bertani (LB)-Medium (1 % Bacto-Trypton, 0,5 % Bacto-Hefeextrakt, 1 % Natriumchlorid, pH 7,0), welches nach dem Autoklavieren mit 100 µg/ml Ampicillin versetzt wird, 16 Stunden bei 37°C angezüchtet. Die Isolierung der Plasmide pJDM1018 und pKMW1 aus diesen Stämmen erfolgt durch alkalische Lyse und anschließende zweimalige Cäsiumchlorid-Dichtegradientenzentrifugation wie in J. Sambrook et al. (1989) beschrieben.

Jeweils 10 µg der isolierten Plasmid-DNA werden in 10 mM Tris-HCI pH 8,0, 5 mM MgCl₂, 100 mM NaCI, 1 mM Mercaptoethanol mit jeweils 5 U EcoRI und Hindlll (Fa. Boehringer Mannheim, Mannheim, Deutschland) versetzt und zwei Stunden bei 37°C inkubiert.

Die gespaltene Plasmid-DNA wird jeweils auf ein horizontales 0,8 %iges Agarosegel aufgetragen und elektrophoretisch aufgetrennt (J. Sambrook et al. 1989).

Mit Hilfe eines Skalpells wird eine schmale Tasche unmittelbar vor den 0,7 kb bzw. 1,2 kb EcoRI/HindIII Fragmenten der beiden Plasmide pJDM1018 und pKMW1 aus dem Agarosegel herausgeschnitten und mit TBE-Puffer (0,045 M Tris-Borat, 0,001 M EDTA, pH 8,0) gefüllt. Die DNA-Fragmente werden elektrophoretisch wie in T. Maniatis et al. (1989) beschrieben in die Tasche transferiert. Der Puffer wird daraus entnommen, mit Phenol/Chloroform und Chloroform/Isoamylalkohol extrahiert und anschließend mit absolutem Ethanol gefällt, mit 70 % Ethanol gewaschen, das erhaltene DNA-Sediment getrocknet und in 50 µl TE-Puffer (10 mM Tris-HCI, 0,1 mM EDTA, pH 8,0) aufgenommen.

### 2. Herstellung der Gensonden strD und strE

5 µg der nach Punkt 1 hergestellten DNA-Fragmente der Plasmide pJDM1018 (strD-Gen) und pKMW1 (strE-Gen) werden in 50 mM Tris-HCI, pH 7,5, 10 mM MgSO₄, 0,1 mM Dithiothreitol, 50 µg/ml Rinderserumalbumin (Fraktion V, Firma Sigma, Deisenhofen, Deutschland) mit 5 U E. coli DNA-Polymerase I (Nick translation grade, Boehringer Mannheim, Deutschland) und 50 µM dATP, 50 µM dTTP und jeweils 40 µ Ci[α^{-32P}]-dCTP und [α^{-32P}]-dGTP (3000 Ci/mMol; Fa. DuPont de Nemours, NEN Division, Dreieich, Deutschland) 35 Minuten bei 15°C inkubiert. Die Reaktion wird durch Zugabe von 0,02 mM EDTA pH 8,0 und 10 Minuten Erhitzen auf 65°C beendet. Nicht eingebaute Radionuklide werden wie in J. Sambrook (1989) beschrieben durch Gelfiltration an einer
0,5 x 10 cm Sephadex G15-Säule in TE-Puffer (10 mM Tris-HCI, 0,1 M EDTA, pH 8,0) abgetrennt.

Die durch Gelfiltration gereinigten radioaktiv markierten 0,7 kb bzw. 1,2 kb EcoRI/Hindlll-Fragmente der Plasmide pJDM1018 und pKMW1 werden als strD und strE Gensonden bezeichnet und unmittelbar vor Gebrauch mit 0,3 M Natronlauge 5 Minuten bei Raumtemperatur denaturiert, anschließend mit 0,3 M HCI und 0,3 Tris-HCI pH 7,0 neutralisiert, 5 Minuten gekocht und in Eiswasser sofort abgekühlt.

### 3. Isolierung, Spaltung und gelelektrophoretische Auftrennung von Gesamt-DNA aus Streptomyceten

Die Streptomycetenstämme S. nodosus DSM40109 und NRRLB-2371, S. noursei DSM40635 und NRRLB-1714, S. aminophilus DSM40186, S. lucensis DSM40317, S. venezulae NRRLB-2447, S. narbonensis DSM40016, S. griseus DSM40236 und S. glaucescens DSM40716 werden in 100 ml CASO-Bouillon (Caseinpepton-Sojamehlpepton-Bouillon, Fa. Merck, Darmstadt, Deutschland) drei Tage bei 30°C angezüchtet. Die Gesamt-DNA dieser Stämme wird nach der in D.A. Hopwood et al. (1985), Genetic manipulation of Streptomyces; A laboratory manual; The John Innes Foundation, Norwich, England, beschriebenen Methode isoliert.

Die Isolierung der genomischen DNA aus S. purpurascens DSM2658 erfolgt aus Protoplasten, welche nach D.A. Hopwood et al. (1985) präpariert und bei -20°C eingefroren werden.
Die Protoplasten des Stammes S. purpurascens DSM2658 werden bei 37°C rasch aufgetaut und in einer Tischzentrifuge (Z 231 M, Fa. Hermle) 7 Minuten bei 3000 U/Min zentrifugiert. Die sedimentierten Protoplasten werden in 10 mM Tris-HCI, 5 mM EDTA, pH 7,8, 0,5 % SDS und 0,2 mg/ml Proteinase K eine Stunde bei 60°C lysiert. Der Ansatz wird mit 5 mM EDTA, pH 8,0, 1 % SDS, 1 M NaCl 2 Stunden auf Eiswasser gestellt. Nach 30 Min. Zentrifugation bei 10.000 U/Min bei 4°C wird der Überstand in ein 1,5 ml Eppendorf-Reaktionsgefäß übergeführt, dessen Deckel mit Hilfe eines glühenden Spatels durchbohrt wurde. Zwischen Öffnung und Deckel des Reaktionsgefäßes wird eine Dialysemembran gespannt, der Deckel geschlossen und mit Parafilm abgedichtet. Dialyse erfolgt zweimal 5 Stunden bei 4°C in 0,5 Liter 10 mM Tris-HCI, pH 7,5.

10 µg isolierter Gesamt-DNA der untersuchten Streptomyceten-Stämme werden mit 10 U BamHI und 1 U DNase-freier RNase aus Rinder-Pankreas (Boehringer Mannheim, Deutschland), in 10 mM Tris/HCI, pH 8,0, 5 mM MgCl₂, 100 mM NaCl und 1mM Mercaptoethanol zwei Stunden bei 37°C inkubiert.

Die gespaltene genomische DNA der Streptomyceten und die Molekulargewichtsstandards (Hindlll und EcoRI/HindIII-Fragmente der λ-DNA, Boehringer Mannheim) werden auf einem 0,8 % Agarosegel aufgetrennt und anschließend unter UV-Beleuchtung (254 nm) photographiert.

### 4. DNA-Transfer auf Membranen (Southern Transfer)

Die Übertragung von DNA-Fragmenten aus Agarosegelen auf Membranen erfolgt im wesentlichen nach der von E.M. Southern (1975) J. Mol. Biol. 98: 503-517 beschriebenen Methode. Das nach Punkt 3 erhaltene Agarosegel wird 15 Minuten in 0,24 M Salzsäure geschwenkt und anschließend 20 Minuten mit 0,4 M Natronlauge behandelt. Das Gel wird auf 2 Lagen saugfähiges Papier (Whatman 3MM-Chr, Firma Whatmann International Ltd., Maidstone, England) gelegt und eine Hybond™-N+-Membran (Fa. Amersham, Braunschweig, Deutschland) luftblasenfrei darauf ausgebreitet. Wie in J. Sambrook et al. (1989) beschrieben, werden mehrere Stapel saugfähiges Papier darauf geschichtet. Auf den Filterpapierstapel wird ein ca. 1 kg schweres Gewicht gestellt. Der DNA-Transfer erfolgt durch Durchsaugen von 0,4 M Natronlauge. Nach 16 Stunden Transferzeit wird der Nylonfilter mit 0,9 M Natriumchlorid, 0,09 M Tri-Natriumcitrat kurz abgespült und 2 Stunden bei 80°C in einem Trockenschrank gebacken.

### 5. DNA-Hybridisierung und Autoradiographie

Der Nylon-Filter, welcher wie unter Punkt 4 beschrieben behandelt ist, wird zwei Stunden in 50 ml Prähybridisierungslösung (0,9 M Natriumchlorid; 0,09 M Tri-Natriumcitrat, 0,5 % SDS, 20 µg/ml denaturierte Heringssperm-DNA, 0,1 % Rinderserumalbumin (Fraktion V, Sigma), 0,1 % Ficoll (Type 400, Fa. Sigma, 0,1 % Polyvinylpyrrolidon, MW = ca. 40.000 d, Fa. Sigma) bei 68°C geschwenkt. Die Prähybridisierungslösung wird abgegossen und 50 ml Hybridisierungslösung, welche die denaturierte strD bzw. strE-Gensonde in Prähybridisierungslösung ohne Heringssperm-DNA enthält, zugesetzt. Die Hybridisierung erfolgt unter leichtem Schütteln 16 Stunden bei 68°C. Die Filter werden 15 Minuten in 0,3 M Natriumchlorid, 0,03 M Tri-Natriumcitrat und 0,5 % SDS bei 68°C und anschließend dreimal 15 Minuten in 0,075 M Natriumchlorid, 0,0075 M Tri-Natriumcitrat und 0,5 % SDS bei 68°C gewaschen. Die Filter werden bei Raumtemperatur getrocknet, auf ein Whatman 3MM-Chr Filterpapier gelegt und mit Frischhaltefolie abgedeckt. Autoradiographie erfolgt mit Kodak X-Omat AR-Röntgenfilmen in einer lichtdichten Kasette mit Verstärkerfolien bei -80°C mindestens 16 Stunden.

**Tabelle 1**

| Hybridisierung von BamHI-Fragmenten der Gesamt-DNA verschiedener Aktinomyceten mit ³²P-markierten strD, strE-Gensonden. | | | |
|---|---|---|---|
| Gesamt-DNA aus | Produzent von | Hybridisierende BamHI-Fragmente (kb) mit Gensonde | |
| | | strD | strE |
| S. nodosus DSM40109 | Amphotericin A, B | 2,6 | 2,4 |
| | | | 4,4 |
| S. nodosus NRRLB-2371 | Amphotericin A, B | 2,6 | 2,4 |
| | | | 4,4 |
| S. noursei DSM40635 | Nystatin | 3,0 | 3,0 |
| | | | 2,2 |
| S. noursei NRRLB-1714 | Nystatin | 3,0 | 3,0 |
| | | | 2,2 |
| S. aminophilus DSM40186 | Perimycin | 7,0 | 7,0 |
| | | | 3,5 |
| S. lucensis DSM40317 | Lucensomycin | 1,0 | 11,0 |
| S. venezuelae NRRLB-2447 | Methymycin | | 9,0 |
| S. narbonensis DSM40016 | Narbomycin | 5,0 | 5,0 |
| | | 4,0 | |
| S. purpurascens DSM2658 | Rhodomycin | 5,7 | 5,7 |
| S. griseus DSM40236 | Streptomycin | | 9,0 |
| S. glaucescens DSM40716 | Hydroxystreptomycin | 5,0 | 5,0 |
| S. = Streptomyces | | | |

### 6. Isolierung und Klonierung von BamHI-Fragmenten aus der Gesamt-DNA von Streptomyces nodosus DSM40109

Die Gesamt-DNA aus S. nodosus DSM40109 wird isoliert, mit BamHI vollständig gespalten und durch Agarose-Gelelektrophorose aufgetrennt (s. Beispiel 3). BamHI-Fragmente der Länge 1,5 kb bis 3 kb werden aus dem Gel isoliert (s. Beispiel 1).

Das Vektorplasmid wird aus dem Stamm S. coelicolor Müller DSM4914 pEB15 nach der in D.A. Hopwood et al. (1985) S. 85 ff. beschriebenen Methode isoliert, mit Bglll gespalten und mit alkalischer Phosphatase (Calf Intestinal Phosphatase, Boehringer Mannheim, Deutschland) nach der in EP 0 368 224 (Beispiel 2) beschriebenen Methoden behandelt. Jeweils 1 µg der mit Bglll gespaltenen dephosphorylierten Vektor-DNA wird in einem 20 µl Reaktionsansatz mit 5 µg isolierten 1,5 kb bis 3 kb BamHI-Fragmenten der Gesamt-DNA von S. nodosus DSM40109 mit 1 U T4-DNA-Ligase (Boehringer Mannheim) 16 Stunden bei 16°C in Ligase Puffer (66 mM Tris-HCl, pH 7,5, 5 mM MgCl₂, 1 mM Dithiothreitol, 1 mM ATP) inkubiert. Protoplasten von S. sp. DSM40434 werden nach D.A. Hopwood et al. Seite 12 ff. hergestellt. Der Ligase-Ansatz wird wie in Hopwood et al. Seite 110 ff. beschrieben, in Protoplasten von S. lividans DSM40434 transformiert. Thiostrepton-resistente Transformanden werden in 2,5 ml CASO-Boullion (Merck, Darmstadt), welche mit 30 µg/ml Thiostrepton versetzt wird, 3 Tage bei 30°C angezüchtet. Die Plasmid-DNA wird nach der in Hopwood et al. Seite 85 ff. beschriebenen Methode isoliert, mit Clal geschnitten und gelelektrophoretisch aufgetrennt. Mind. 30 % der untersuchten Transformanden weisen ein Plasmid der Größe 6,8 kb bis 8,3 kb auf.

### 7. Identifizierung von Klonen, welche dTDP-D-Glukose-Synthasegene enthalten

Jeweils zehn der nach Beispiel 6 erhaltenen Klone werden in 2,5 ml CASO-Bouillon mit 30 µg/ml Thiostrepton drei Tage bei 30°C angezüchtet. Die Plasmid-DNA aus insgesamt 200 dieser sogenannten Zehner-Pools wird isoliert (Hopwood Seite 85 ff.). Jeweils 5 µl der isolierten Plasmid-DNA wird 10 Minuten auf 96°C erhitzt und rasch in Eiswasser abgekühlt. 3 µl der denaturierten Plasmid-DNA jedes Pools wird in einem regelmäßigen Muster mit jeweils 1,5 cm Abstand auf eine Membran (Hybond™-N⁺, Amersham Buchler, Braunschweig, Deutschland) aufgetragen. Nach dem Trocknen bei Raumtemperatur wird die Membran 5 Minuten auf ein mit Denaturierungspuffer I (1,5 M NaCl, 0,5 M NaOH) getränktes Filterpapier (Whatman 3MM-Chr, Whatmann International Ltd. Maidstone, England) gelegt. Anschließend wird 1 Min. bzw. 20 Min. ein mit Neutrallösung (1,5 M NaCl, 0,5 M Tris-HCl, pH 7,2, 1 mM EDTA) bzw. Denaturierungspuffer II (0,4 M NaOH) getränktes Filterpapier eingesetzt. Die Hybond™-N⁺-Membran wird mit 5-fach konzentrierter SSC-Lösung (entspricht 0,75 M Natriumchlorid, 0,075 M Tri-Natriumcitrat, pH 7,5) abgespült und anschließend wie in Beispiel 5 beschrieben mit der strD-Sonde (s. Beispiel 2) hybridisiert. 3 von 200 Zehnerpools hybridisieren mit der eingesetzten Sonde. Ein ausgewählter Pool wird in die zehn Einzelklone aufgesplittet, deren Plasmid-DNA isoliert und wie in Beispiel 5 beschrieben mit der strD-Sonde (s. Beispiel 2) hybridisiert. Das hybridisierende Plasmid wird mit pPS72.2 bezeichnet (s. Abb. 3B). Es enthält ein 2,6 kb BamHI-Fragment in den Vektor pEB15 kloniert.

### 8. Subklonierung des Plasmids pPS72.2

Das Plasmid pPS72.2 wird durch Cäsiumchlorid-Dichtegradienten-Zentrifugation aus dem nach Beispiel 6 und 7 isolierten S. lividans-Klon isoliert (Hopwood et al., S. 87, 82, 93). Das Plasmid wird mit Smal und Sstl gespalten und das erhaltene 1,4 kb Smal/Sstl-Fragment aus dem Gel isoliert. Das Vektorplasmid pUC18 wird von Boehringer Mannheim, Deutschland, bezogen, mit Smal und Sstl geschnitten und das 2,7 kb Fragment isoliert. Jeweils 1 µg der isolierten 1,4 kb und 2,7 kb Fragmente werden mit T4-DNA-Ligase behandelt und in kompetente Zellen von E. coli DH5Alpha (MAX Efficiency DH5Alpha™ competent cells, Gibco BRL, Eggenstein, Deutschland) nach den Angaben des Herstellers transformiert und auf LB-Platten (s. Beispiel 1) mit 100 µg/ml Ampicillin 16 Stunden bei 37°C selektioniert. Ampicillin-resistente Kolonien werden in 2,5 ml LB-Medium mit 100 µg/ml Ampicillin 16 Stunden bei 37°C angezüchtet und die Plasmid-DNA durch alkalische Minilyse (J. Sambrook et al. 1989, 1.25 bis 1.28) isoliert, mit Smal und Sstl verdaut und gelelektrophoretisch aufgetrennt. Ein Plasmid, welches ein 1,4 kb Smal/Sstl-Fragment enthält, wird als pPS1 bezeichnet (s. Abb. 3B).

Das Plasmid pPS72.2 wird mit Smal gespalten und das 1,45 Smal-Fragment isoliert. Mit Smal linearisierter, dephosphorylierter Vektor pUC18 wird von Boehringer Mannheim, Deutschland, bezogen. Jeweils 1 µg des isolierten 1,45 kb Smal-Fragmentes von pPS72.2 wird mit den Smal linearisierten, dephosphorylierten Vektor pUC18 mit T4-DNA-Ligase verknüpft und in kompetente Zellen von E. coli DH5Alpha transformiert. Plasmid-DNA aus erhaltenen Ampicillin-resistenten Klonen wird isoliert, mit EcoRI geschnitten und gelelektrophoretisch aufgetrennt. Ein Plasmid, welches ein 0,4 kb EcoRI-Fragment aufweist, wird als pSab1 bezeichnet und enthält das 1,45 kb Smal-Fragment in der in Abb. 3B gezeigten Orientierung in pUC18 kloniert.

Das Plasmid pSab1 wird mit EcoRI verdaut. Das erhaltene 3,7 kb Fragment wird isoliert, mit T4-DNA-Ligase religiert und in E. coli DH5Alpha transformiert. Das entstandene 3,7 kb Plasmid wird als pSab3 bezeichnet (s. Abb. 3B).

Das Plasmid pSabl wird mit EcoRI gespalten, das 0,4 kb EcoRI-Fragment isoliert, mit EcoRI linearisiertem, dephosphoryliertem Vektor pUC18 ligiert und in kompetente Zellen von E. coli DH5Alpha transformiert. Plasmid-DNA enthaltende Ampicillin-resistente Transformanden werden isoliert und mit Smal gespalten. Ein Plasmid, welches ein 0,4 kb Smal-Fragment aufweist, wird als pSab2.1 bezeichnet (s. Abb. 3B). Ein Plasmid, welches ein 3,1 kb Smal-Fragment enthält, wird pSab2.2 genannt (s. Abb. 3B).

### 9. DNA-Sequenzanalyse des 2,6 kb BamHI-Fragmentes von S. nodosus DSM40109

Die Plasmide pPS72.2, pPS1, pSab1, pSab2.1, pSab2.2 und pSab3 (s. Abb. 3A,B) werden nach alkalischer Lyse zweimal durch Cäsiumchlorid-Dichtegradientzentrifugation gereinigt (s. J. Sambrook et al., 1989, 1.38 bis 1.43).

Zwei Primer (pUC, sequencing und reverse sequencing) werden von Boehringer, Mannheim, Deutschland, bezogen. Die anderen in Tabelle 2 aufgeführten Primer werden, wie in der europäischen Offenlegungsschrift 0 368 224, Beispiel 16, beschrieben synthetisiert. Die Sequenzen der Primer, die für die Sequenzierung der Plasmide pPS72.2, pPS1, pSab1, pSab2.1, pSab2.2 und pSab3 benutzt wurden, sind in Tabelle 2 zusammengefaßt.

Die Sequenzierung der Doppelstrang-DNA des 2,6 kb BamHI-Fragmentes von S. nodosus DSM40109 erfolgte mit dem Promega fmol™ Sequencing System (Serva, Heidelberg, Deutschland) mit 6 µCi [³⁵S]-Desoxyadenosin-5'-[Alpha-Thio]-Triphosphat (1422 µCi/mmol), DuPont de Nemours, NEN Division, Dreieich, Deutschland.

Die sogenannte Annealing-Temperatur (T_{A}), bei welcher der jeweilige Primer an die Template-DNA anbindet, ist in Tabelle 2 aufgeführt. Denaturierung, Primer-Anbindung (Annealing) und Taql-Polymerase-Reaktion (Elongation) werden in einem Perkin Elmer Cetus DNA Thermal Cycler, Bodenseewerk, Überlingen, Deutschland, durchgeführt. Dabei wird nach 2 Min. Aufheizzeit auf 95°C das Temperaturprogramm 1 (30 Sek. 95°C, 30 Sek. T_{A}, 1 Min. 70°C) bzw. das Temperaturprogramm 2 (30 Sek. 95°C, 30 Sek. 70°C) in jeweils 30 Zyklen, wie in Tabelle 2 angegeben, durchlaufen.

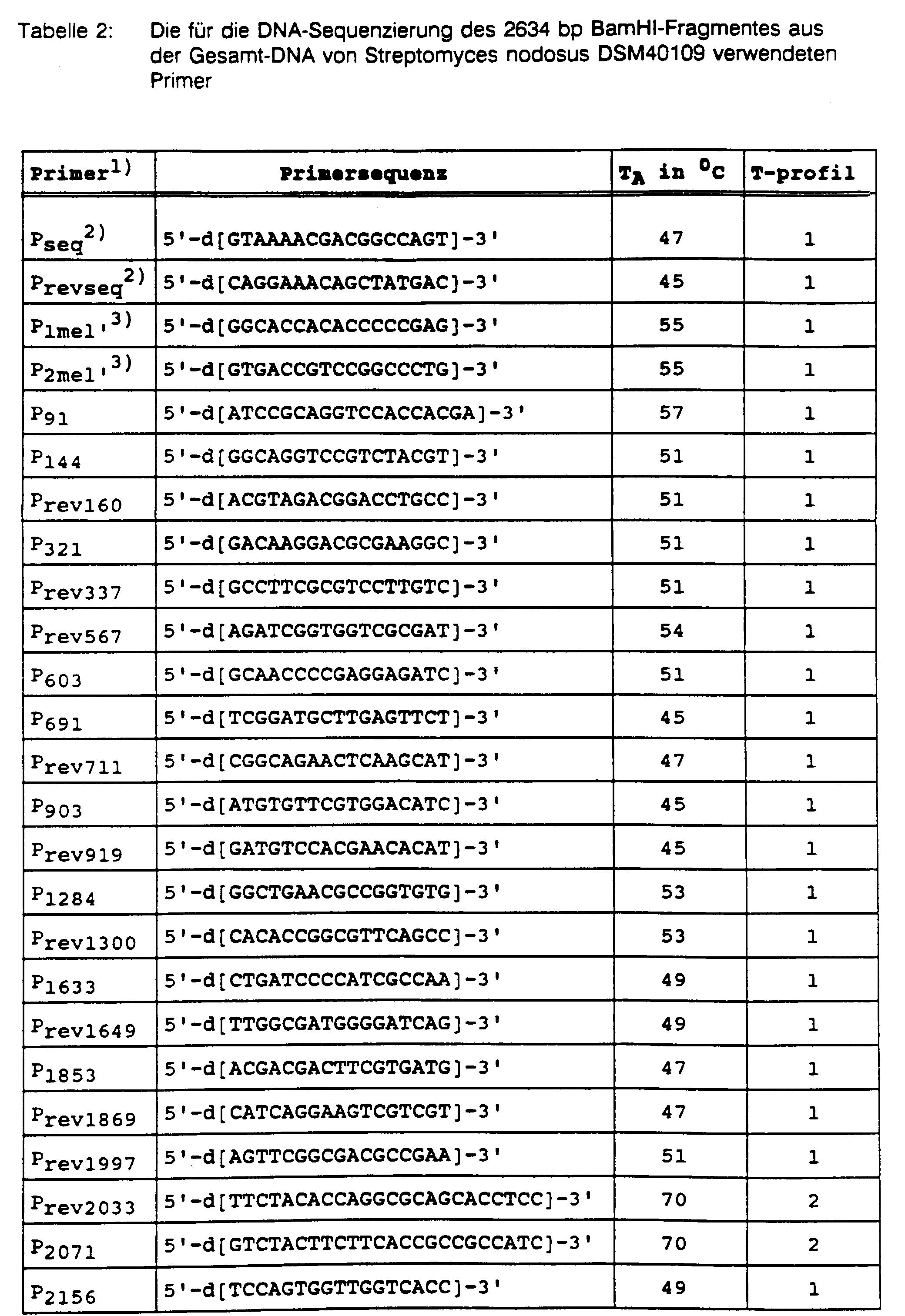

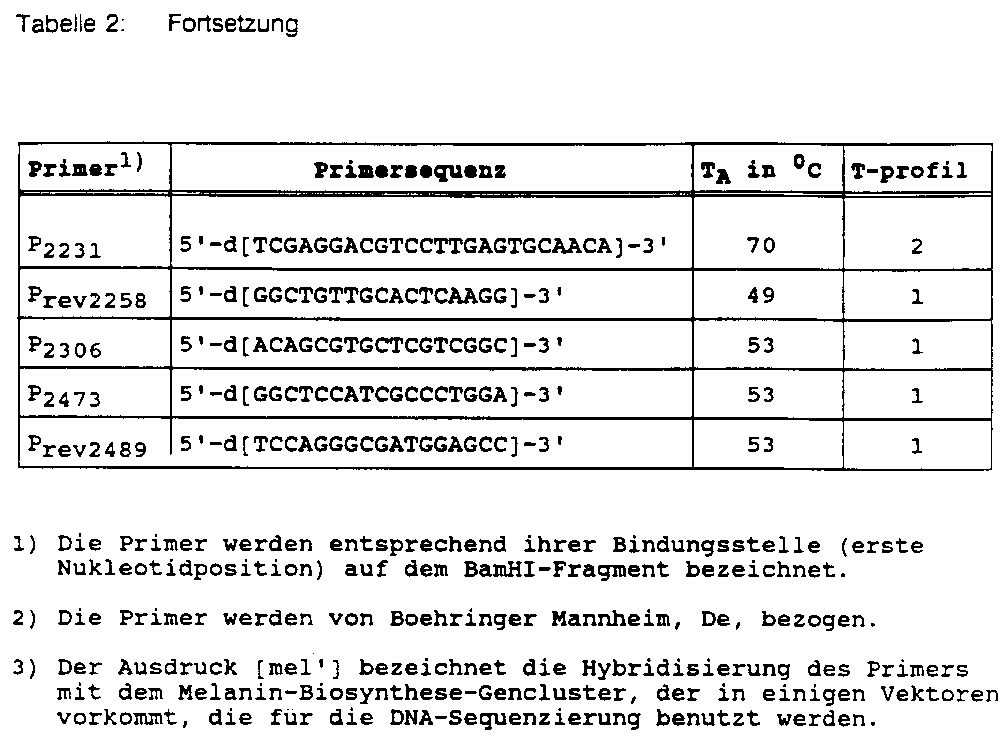

Die vier Probenansätze werden nach Zugabe von Stop-Lösung (s. fmol^{Tm} DNA Sequencing System) auf einem 6 % Polyacrylamid-Harnstoffgel aufgetrennt. Dazu wird die Macrophor Sequenzierkammer, Pharmacia Biosystems GmbH, Freiburg, Deutschland, verwendet. Die Glasplatten werden mit absolutem Ethanol vorgereinigt. Die ausgeschnittene Glasplatte wird mit 8 ml absolutem Alkohol, welcher mit 240 µl 10 % Essigsäure und 40 µl Bind-Silan (Pharmacia) versetzt wird, abgerieben. Die Thermoplatte wird mit 5 ml Repel-Silan (Pharmacia) behandelt. Anschließend wird jeweils mit absolutem Ethanol nachpoliert.

Das 6 % Polyacrylamid-Harnstoffgel besteht aus 6 % Acrylamid/Bisacrylamid (19:1), 7 M Harnstoff in TBE [0,1 M Tris, 89 mM Borsäure, 1 mM EDTA (Ethylendinitrotetraacetat)]. Für die Polymerisation wird 0,06 % Ammoniumpersulfat und 0,1 % TEMED (N,N,N',N'-Tetra-methyl-Ethylen-Diamin) zugesetzt. Vor dem Gießen wird die Lösung durch eine Cellulosenitratmembran (Nalgene® Typ S, Porendurchmesser 0,2 µm, Oskar Glock, Offenbach) filtriert und entgast.

Die Auftrennung erfolgt in TBE-Puffer für 1,5 Std. oder 3,5 Std. bei 2400 V bei 60°C. Das Gel wird anschließend 10 Min. in 10 % Essigsäure geschwenkt, anschließend mit Wasser kurz abgespült und 30 Min. bei 65°C getrocknet. Autoradiographie erfolgt mit Kodak X-Omat™ XAR5 Filmen in einer Expositionskassette mit Verstärkerfolien (Dr. Goos Suprema Universal) 1 bis 3 Tage bei Raumtemperatur. Der Röntgenfilm wird in einer Entwicklermaschine (Agfa Gevaert) entwickelt.

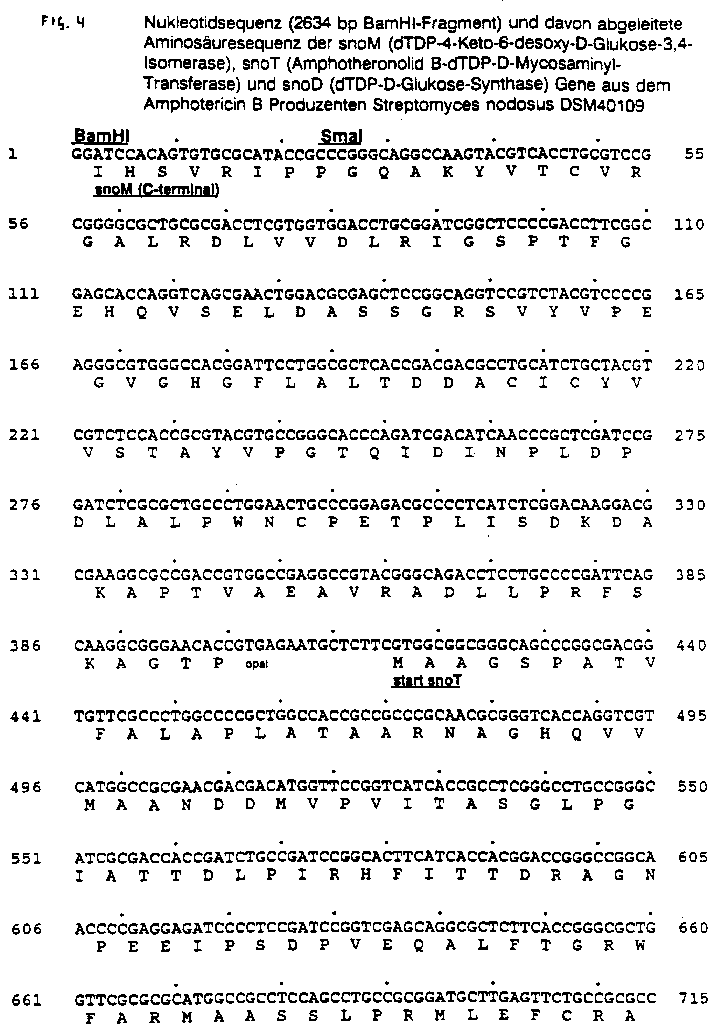

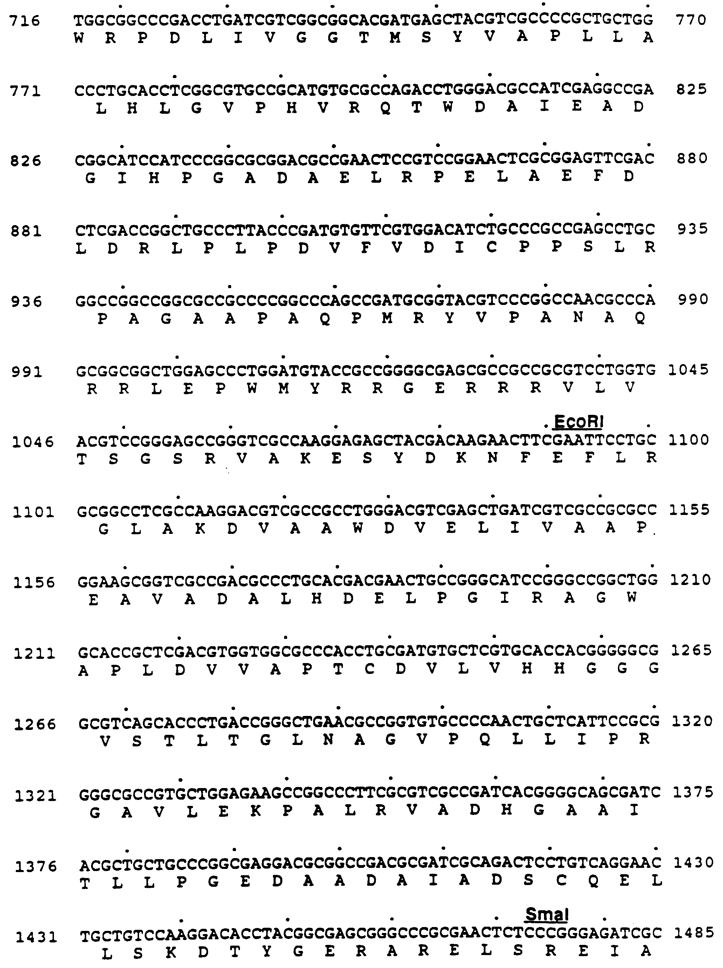

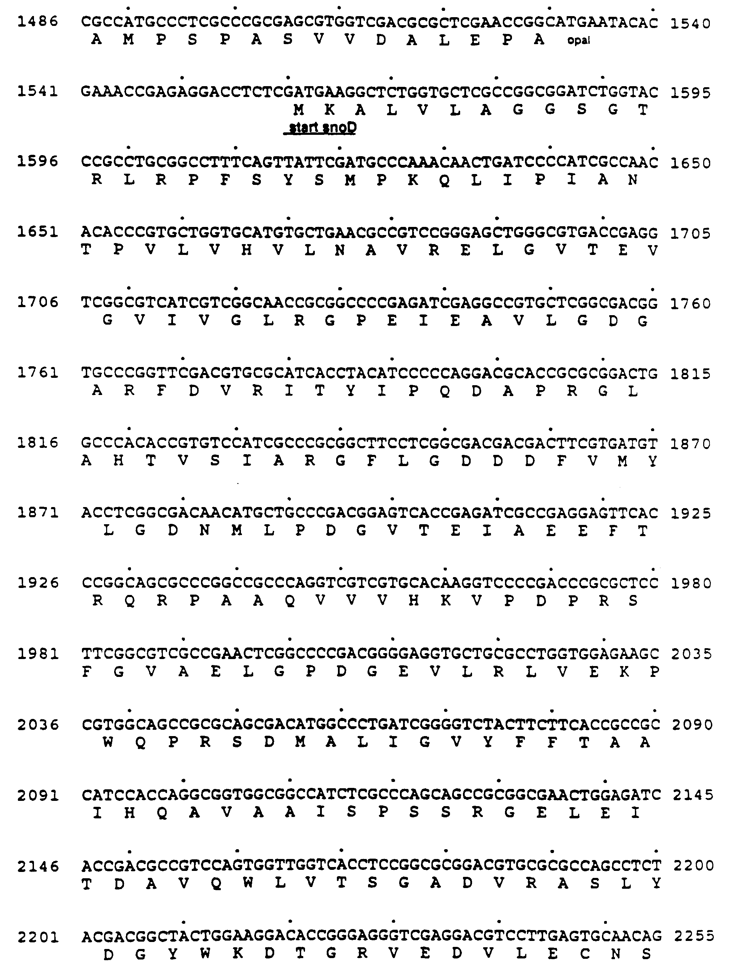

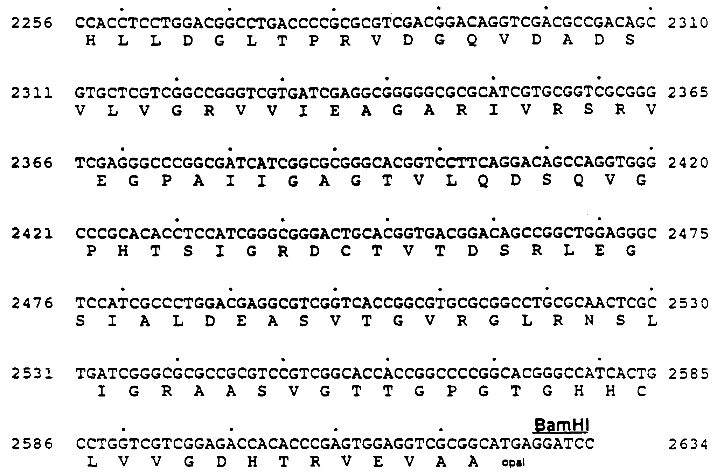

Die DNA-Sequenz des 2634 kb BamHI-Fragmentes ist in Abb. 4 dargestellt. Die erhaltene DNA-Sequenz wird von Nukleotid-Position Nr. 1 bis 401 in die entsprechende Aminosäureabfolge übersetzt. Die Codon-Verwendung in diesem Abschnitt (snoM-Genabschnitt) entspricht dem für Streptomyceten-Gene charakteristischen Codon-Gebrauch (F. Wright et al., 1992, Gen 113:55-65). An Nukleotid-Position Nr. 402 befindet sich ein Stop-Codon (TGA). Der identifizierte offene Leserahmen umfaßt 133 Aminosäurereste. Durch Vergleich dieser Aminosäuresequenz mit der EMBL-Datenbank (TFASTA-Programm des Database Searching Programme des GCG-Package, Version 7, Genetics Computer Group Inc. Madison, Wisconsin, USA) wird eine 42 %ige Übereinstimmung mit dem C-terminalen Teil (Aminosäure Position Nr. 59 bis 201) der dTDP-4-Keto-6-Desoxy-D-Glukose-3,5-Epimerase (strM) von S. griseus DSM40236 festgestellt. Aus dem Vergleich der Biosynthesewege von dTDP-L-Dihydrostreptose (Abb. 1) und dTDP-D-Mycosamin (Abb 3 und 4.4) wird daher das snoM-Genprodukt als dTDP-4-Keto-6-Desoxy-D-Glukose-3,4-lsomerase bezeichnet.

Ein weiterer offener Leserahmen (snoT-Genabschnitt) befindet sich von Nukleotid-Position Nr. 416 bis 1532. Die abgeleitete Aminosäuresequenz umfaßt 372 Reste. In dem Bereich von Aminosäureposition Nr. 259 bis 340 liegt 29 % Übereinstimmung mit dem entsprechenden Teil (Aminosäure-Position Nr. 329 bis 412) der Flavonol-O-3-Glukosyl-Transferase von Mais vor. Daher wird das snoT-Genprodukt als Amphoteronolid B-dTDP-Mycosaminyl-Transferase bezeichnet.

Ein weiterer offener Leserahmen (snoD-Genabschnitt) befindet sich von Nukleotid-Position Nr. 1561 bis 2625. Das entsprechende Genprodukt (355 Aminosäurereste) weist 54 % Sequenzidentität mit der dTDP-D-Glukose-Synthase aus S. griseus DSM40236 auf und wird daher als dTDP-D-Glukose-Synthase bezeichnet (s. Abb. 1 und Abb. 3 und 4.4).

## Patentansprüche

1. DNA-Sequenz der Abb. 4.

2. DNA-Sequenz nach Anspruch 1, dadurch gekennzeichnet, daß die vollständige snoT-DNA-Sequenz, kodierend für die Amphotheronolid B-dTDP-D-Mycosaminyl-Transferase, die vollständige snoD-DNA-Sequenz, kodierend für die dTDP-D-Glukose-Synthase und die DNA-Teilsequenz von snoM, kodierend für die dTDP-4-Keto-6-Desoxy-D-Glukose-lsomerase, enthalten sind.

3. Aminosäuresequenz der Abb. 4.

## Claims

1. The DNA sequence of Fig. 4.

2. The DNA sequence as claimed in claim 1, which contains the complete snoT DNA sequence, encoding amphotheronolide B-dTDP-D-mycosaminyl transferase, the complete snoD DNA sequence, encoding dTDP-D-glucose synthase, and part of the DNA sequence of snoM, encoding dTDP-4-keto-6-deoxy-D-glucose isomerase.

3. The amino acid sequence of Fig. 4.

## Revendications

1. Séquence d'ADN de la figure 4.

2. Séquence d'ADN selon la revendication 1, caractérisée en ce que la séquence complète d'ADN snoT codant pour l'amphothéronolide B-dTDP-D-mycosaminyl-transférase, la séquence complète d'ADN snoD codant pour la dTDP-D-glucose-synthase et la séquence partielle d'ADN de snoM codant pour le dTDP-4-céto-6-désoxy-D-glucose-isomérase.

3. Séquence d'acides aminés de la figure 4.
